(19) Europäisches Patentamt
European Patent Office
Office européen des brevets

(11) Publication number: **0 520 160 A1**

# (12) EUROPEAN PATENT APPLICATION

(21) Application number: **92107452.2**

(22) Date of filing: **30.04.92**

(51) Int. Cl.[5]: **A61L 29/00**, A61L 27/00, A61L 31/00, A61L 15/44, A61L 15/26

(30) Priority: **28.06.91 US 722784**

(43) Date of publication of application: **30.12.92 Bulletin 92/53**

(84) Designated Contracting States: **AT BE CH DE DK ES FR GB IT LI LU NL PT SE**

(71) Applicant: **BOC HEALTH CARE, Inc.**
**575 Mountain Avenue**
**Murray Hill, New Jersey 07974(US)**

(72) Inventor: **Laufer, Jay K.**
**13901 Orange Dale Place**
**Tampa, Florida 33625(US)**

(74) Representative: **Dipl.-Phys.Dr. Manitz Dipl.-Ing., Dipl.-W.-Ing. Finsterwald Dipl.-Ing. Grämkow Dipl.-Chem.Dr. Heyn Dipl.-Phys. Rotermund**
**Morgan, B.Sc.(Phys.) Robert-Koch-Strasse 1**
**W-8000 München 22(DE)**

## (54) Process for antimicrobial treatment of polyurethane.

(57) The present invention is directed to a method for impregnating a preformed polyurethane medical device with an antimicrobial quaternary ammonium compound which comprises the steps of (A) contacting said device with a solution containing said compound in a chlorinated or fluorinated hydrocarbon solvent thereby impregnating the device, and (B) removing the solvent, wherein the antimicrobial quaternary ammonium compound is represented by Formula (I):

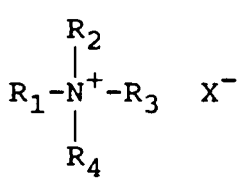

(I)

including optically active isomeric forms thereof, wherein (a) $R_1$, $R_2$, $R_3$, and $R_4$ are independently selected from the group consisting of straight- or branched-chain, saturated or unsaturated alkyl groups having from 1 to 20 carbon atoms, or (b) $R_1$, $R_2$, and $R_3$ are as defined above and $R_4$ is selected from the group consisting of aryl alkyl, chloroaryl alkyl, aryloxy alkyl, and trialkyl ammonium alkyl, or (c) $R_1$ and $R_2$ are as defined above and $R_3$ and $R_4$ are independently selected from the group consisting of aryl alkyl, monoalkylaryloxy alkyleneoxy alkyl, and dialkylaryloxy alkyleneoxy alkyl, or (d) $R_1$ is as defined above and $R_2$, $R_3$, $R_4$, and the nitrogen atom are members of a heterocyclic ring, and $X^-$ is a halogen.

EP 0 520 160 A1

Rank Xerox (UK) Business Services

## BACKGROUND OF THE INVENTION

### 1. Field of the Invention

The present invention is directed to a method for impregnating a polyurethane medical device with an antimicrobial compound. More particularly, the present invention is directed to a method for impregnating a polyurethane medical device with a solution of an antimicrobial quaternary ammonium compound in a chlorinated or fluorinated hydrocarbon solvent and removing the solvent.

### 2. Description of the Prior Art

Medical devices formed from polyurethane polymers are currently used for many invasive procedures to treat a variety of diseases. Such invasive devices include intravenous and urinary catheters, test probes, peristaltic pump chambers, implant materials, arteriovenous shunts, gastroenteric feed tubes, endotracheal tubes, and the like. These invasive devices can provide access from the environment into a patient's body thereby increasing the risk of mycotic and bacterial infection. One method for preventing such infection is to provide an antimicrobial barrier on the medical device which prevents the migration of microorganisms along the device. Medical devices which may employ such antimicrobial barriers include films for burn and wound dressings, sponges for wound cleansing, condoms, and the like. A disadvantage of conventional antimicrobial barriers on medical devices is that such barriers are generally applied as surface coatings which tend to be rapidly leached away and thus do not provide prolonged antimicrobial protection.

United States patent no. 4,479,795, issued to Mustacich et al., discloses a method for preparing a polymeric catheter have releasably incorporated therein an effective amount of a free *n*-alkane monocarboxylic acid antimicrobial agent and a non-antimicrobial proton donor. The antimicrobial agent is incorporated into the catheter by either soaking the catheter therein or adding a water-soluble salt of the antimicrobial agent to the catheter polymer in the pre-curing stage.

United States patent no. 4,877,617, issued to Namikoshi et al., discloses a method for imparting fungicidal and bacteriocidal properties to an article which comprises impregnating the article with a solution of a quaternary ammonium salt of alginic acid or carboxymethylcellulose. The solvents employed for the alginic acid or carboxymethylcellulose derivatives are acetone, toluene, and lower-alkyl alcohols such as methanol, ethanol, propanol, and isopropanol. Namikoshi et al. does not disclose the impregnation of polyurethane.

United States patent no. 4,675,347, issued to Mochizuki et al., discloses an antimicrobial latex composition which comprises a cationic latex component which may be a natural rubber latex or a synthetic polymer latex and a cationic antimicrobial agent incorporated into the latex composition. The latex composition may be utilized to form a medical device.

United States patent no. 4,603,152, issued to Laurin et al., discloses an antimicrobial composition which comprises an antimicrobial metal incorporated into a polymer which may be polyurethane.

United States patent no. 4,925,668, issued to Khan et al., discloses a method for preparing a medical article which comprises forming a homogeneous melt of chlorhexidine and a hydrophilic polymer which may be polyurethane and extruding the melt to form the article. The article is coated with a coating comprising chlorhexidine and a silicon lubricant. United States patent no. 4,999,210, issued to Solomon et al., discloses a method for laminating the medical article of Khan et al. which comprises dipping the article into a solution of chlorhexidine and a hydrophilic polymer.

European patent application no. 107,277A1, published May 2, 1984, discloses an antimicrobial polymer comprising a copolymer of vinyl acetate and vinyl pyrrolidone complexed with iodine or bromine.

European patent application no. 405,284A2, published January 2, 1991, discloses a method for impregnating a catheter with a pharmaceutical composition by contacting the catheter with a solution of the pharmaceutical composition at the supercritical pressure and temperature of the solvent.

United States patent no. 4,605,564, issued to Kulla et al., discloses a method for making an antimicrobial medical device which comprises treating a latex device with a solution of *para*-chloro-*meta*-xylenol in a chlorinated organic solvent and evaporating the solvent to impregnate the *para*-chloro-*meta*-xylenol in the device.

United States patent no. 4,769,013, issued to Lorenz et al., discloses a method for making an antimicrobial coating for a medical device which comprises complexing polyvinylpyrrolidone with polyurethane, and complexing an antibacterial agent with the complexed polyvinylpyrrolidone.

United States patent no. 4,950,256, issued to Luther et al., discloses a catheter coated with a hydrophilic polymer having absorbed thereon an effective amount of the antimicrobial anticoagulant

polymyxin.

United States patent no. 4,923,450, issued to Maeda et al., discloses a medical device containing zeolite and an antibacterial metal selected from the group consisting of silver, copper, and zinc.

United States patent no. 4,589,873, issued to Schwartz et al., discloses a method for coating a polymer such as polyurethane with polyvinyl pyrrolidone in a solvent wherein the solvent is selected from the group consisting of dimethyl formamide, butanone, methanol, tetrahydrofuran, and dimethyl acetamide.

While a number of methods have been reported for providing polymeric medical devices with antimicrobial activity, none of these methods are entirely satisfactory. Accordingly, medical devices containing antimicrobial agents which can be slowly released over a prolonged period of time under typical usage conditions would be highly desirable. The present invention provides such improved polyurethane medical devices impregnated with antimicrobial compounds which possess continuous antimicrobial activity for over one week in vivo or longer.

## SUMMARY OF THE INVENTION

The present invention is directed to a method for impregnating a preformed polyurethane medical device with an antimicrobial quaternary ammonium compound which comprises the steps of (A) contacting said device with a solution containing said compound in a chlorinated or fluorinated hydrocarbon solvent thereby impregnating the device, and (B) removing the solvent, wherein the antimicrobial quaternary ammonium compound is represented by Formula (I):

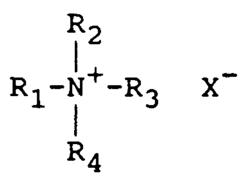

(I)

including optically active isomeric forms thereof, wherein (a) $R_1$, $R_2$, $R_3$, and $R_4$ are independently selected from the group consisting of straight- or branched-chain, saturated or unsaturated alkyl groups having from 1 to 20 carbon atoms, or (b) $R_1$, $R_2$, and $R_3$ are as defined above and $R_4$ is selected from the group consisting of aryl alkyl, chloroaryl alkyl, aryloxy alkyl, and trialkyl ammonium alkyl, or (c) $R_1$ and $R_2$ are as defined above and $R_3$ and $R_4$ are independently selected from the group consisting of aryl alkyl, monoalkylaryloxy alkyleneoxy alkyl, and dialkylaryloxy alkyleneoxy alkyl, or (d) $R_1$ is as defined above and $R_2$, $R_3$, $R_4$, and the nitrogen atom are members of a heterocyclic ring, and $X^-$ is a halogen.

## DETAILED DESCRIPTION OF THE INVENTION

The present invention is directed to a polyurethane medical device having prolonged antimicrobial activity. In accordance with the invention, a preformed medical device is contacted with a solution containing an antimicrobial quaternary ammonium compound in a chlorinated or fluorinated hydrocarbon solvent, and the solvent is thereafter removed. Upon being hydrated, the impregnated polyurethane medical device slowly releases the antimicrobial compound to provide sustained microbial inhibition in the environment of the medical device. The antimicrobial compounds can be impregnated into a wide variety of medical devices.

Applicant has found that by contacting a preformed polyurethane medical device with a solution of an antimicrobial quaternary ammonium compound in a chlorinated or fluorinated hydrocarbon solvent, impregnated devices having prolonged antimicrobial activity can be prepared. While not wishing to be bound by theory, applicant believes that the chlorinated or fluorinated hydrocarbon solvent solvates, swells, and penetrates the polyurethane polymeric matrix of the medical device allowing the molecules of the antimicrobial quaternary ammonium compound to enter therethrough. When the solvent is evaporated, the polyurethane matrix contracts entrapping the dispersed antimicrobial compound. Because polyurethane Is permeable to water, the entrapped antimicrobial molecules slowly dissolve and are leached away from the medical device when it is employed in an invasive procedure. The antimicrobial activity of the impregnated polyurethane device in vivo will continue for up to about one week or longer depending upon the concentration of the antimicrobial quaternary ammonium compound. The concentration of the antimicrobial quaternary ammonium compound in the medical device can be controlled by controlling its concentration in

the impregnating solvent, the time period of impregnation, and the type of impregnating solvent employed.

Polyurethanes are an art recognized group of thermoplastic polymers prepared by the condensation reaction of an aromatic or aliphatic polyisocyanate (such as toluene diisocyanate) and a hydroxyl-containing material such as a phenol, an amine, an hydroxyl or a carboxylic compound to produce a polymer with free isocyanate groups. The basic polyurethane polymer unit is formed by the following type of reaction:

$$R_1NCO + R_2OH \rightarrow R_1NHCOOR_2$$

Useful polyisocyanates in the above reaction include toluene-2,4-diisocyanate, toluene-2,6-diisocyanate, commercial mixtures of toluene-2,4- and 2,6-diisocyanates, cyclohexylene-1,4-diisocyanate, m-phenylene diisocyanate, 3,3-diphenyl-4,4-biphenylene diisocyanate, 4,4-biphenylene diisocyanate, 2,4-diisocyanatodiphenylether, 2,4-dimethyl-1,3-phenylene diisocyanate, 4,4-diisocyanatodiphenylether, 2,4,5-toluene triisocyanate, isophorone diisocyanate, and the like.

Useful polyols in the above reaction include polyester polyols obtained from the condensation of polycarboxylic acids, preferably dicarboxylic acids, such as adipic, sevacic, phthalic, oxalic, malonic, cyclohexane-1,2-dicarboxylic, naphthalene-1,2-dicarboxylic, fumaric, itaconic, and the like, with polyalcohols, preferably diols, such as ethylene glycol, diethylene glycol, sorbitol, triethanolamine, di(betahydroxyethyl)-ether, and the like, and amino alcohols such as ethanolamine, 3-aminopropanol, 10-aminodecanol, 6-amino-5-methylhexanol-1,p-hydroxymethylbenzylamine, and the like. Polyesters derived from ring-opening or condensation of lactones with polyfunctional compounds such as any of the polyalcohols set out above can also be used to provide the thermoplastic polyurethane polymers of the present invention.

Examples of preferred thermoplastic physiologically acceptable polyurethanes in accordance with the present invention include polytetramethylene ether glycol diphenylmethane diisocyanate (MDI), polytetramethylene ether glycol tolylene diisocyanate (TDI), poly(1,4-oxybutylene) glycol diphenylmethane diisocyanate (MDI), poly(1,4-oxybutylene) glycol tolylene diisocyanate (TDI), poly(1,4-oxybutylene) glycol isoferrone isocyanate, polyethylene glycol diphenylmethane diisocyanate (MDI), polypropylene glycol tolylene diisocyanate (TDI), polycaprolactone diphenylmethane diisocyanate (MDI), polyethylene adipate diphenylmethane diisocyanate (MDI), polytetramethylene adipate diphenylmethane diisocyanate (MDI), and polyethylenepropylene adipate isophorone isocyanate polyurethanes, and the like, and mixtures thereof. Preferred physiologically acceptable polyurethanes include Pellethane™ series 2363, commercially available from Dow Chemical Company, Estane™, commercially available from B.F.Goodrich, Texin™, commercially available from Mobay, and Mor-Thane™, commercially available from Morton Thiokol. In a most preferred embodiment, the physiologically acceptable polyurethane is Pellethane™ series 2363. Pellethane™ series 2363, which is a polyether polyurethane synthesized from methylene bis (p-phenyl isocyanate) polytetramethylene glycols and 1,4-butanediol.

Although polyurethanes are generally linear in order to provide solubility and thermoplasticity, they can be crosslinked by adding a sufficient quantity of crosslinking agent to a solvent solution of the polymer or by incorporating the crosslinking agent into a melt polymer mixture while it is still in the plastic state. Examples of such crosslinking agents are isocyanates, polycarboxylic acids, peroxides, organo-titanates, and the like.

Chain extenders with hydrogen containing difunctional compounds such as water, diamines, or amino acids may also be used. Useful chain extenders include 1,4-butanediol, hexamethylene diamine, 4,4-methylene-bis(2-chloroaniline) (MOCA), trimethylolpropane, and ethanolamine. Other useful additives include accelerators, catalysts, stabilizers, plasticizers, and the like, which improve or modify the properties of the polyurethane. Examples of such useful additives include dicumyl peroxide, benzothiazole disulfide, polypropylene adipate, and metal salts such as potassium acetate, cobalt naphthenate, and zinc chloride.

The polyurethane medical device treated in accordance with the present invention may be formed by any conventional method such as by extrusion, casting, injection molding, and the like. The device may be of any desired shape, size, or configuration such as a valve, pin, sleeve, prosthetic device, gloves, and the like. The medical devices include such invasive devices as intravenous and urinary catheters, test probes, peristaltic pump chambers, implant materials, arteriovenous shunts, gastroenteric feed tubes, endotracheal tubes, and the like. Other medical devices include films for burn and wound dressings, sponges for wound cleansing, condoms, and the like.

The term "quaternary ammonium compound" as used herein means a physiologically acceptable salt of a basic organic nitrogen compound in which the molecular structure includes a central nitrogen atom (cation) bonded to four organic groups and an acid radical (anion). The antimicrobial quaternary ammonium compounds are direct counterparts of soaps and possess a combination of ionic and non-ionic properties which produce their antimicrobial properties. A variety of mechanisms have been proposed for this

antimicrobial action such as the inactivation of certain enzymes, the denaturation of protein, and the destruction of cell walls.

The antimicrobial quaternary ammonium compounds of the present invention are represented by Formula (I):

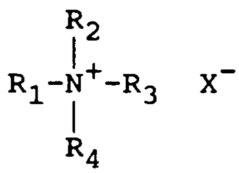

(I)

including optically active isomeric forms thereof, wherein:

(a) $R_1$, $R_2$, $R_3$, and $R_4$ are independently selected from the group consisting of straight- or branched-chain, saturated or unsaturated alkyl groups having from 1 to 20 carbon atoms; or

(b) $R_1$, $R_2$, and $R_3$ are as defined above and $R_4$ is selected from the group consisting of aryl alkyl, chloroaryl alkyl, aryloxy alkyl, and trialkyl ammonium alkyl; or

(c) $R_1$ and $R_2$ are as defined above and $R_3$ and $R_4$ are independently selected from the group consisting of aryl alkyl, monoalkylaryloxy alkyleneoxy alkyl, and dialkylaryloxy alkyleneoxy alkyl; or

(d) $R_1$ is as defined above and $R_2$, $R_3$, $R_4$, and the nitrogen atom are members of a heterocyclic ring; and $X^-$ is a halogen.

Nonlimiting examples of antimicrobial quaternary ammonium compounds in group (a) of Formula (I) include: dodecyl (C-12, lauryl) trimethyl ammonium; hexadecyl (C-16, cetyl) trimethyl ammonium; hexadecyl dimethyl ethyl ammonium; octadecyl (C-18, stearyl) trimethyl ammonium; trioctyl methyl ammonium; dimethyl dioctadecyl ammonium; dimethyl *cis*-9-octadecenyl (C-18, oleyl) 9, 12, 15-octadectrienyl (C-18, linoleyl) ammonium; trimethyl monodocosanyl (C-22, behenyl) ammonium; methyl tridodecyl ammonium; and mixtures thereof. In a preferred embodiment, the antimicrobial quaternary ammonium compound in group (a) is selected from the group consisting of octadecyl trimethyl ammonium, dimethyl dioctadecyl ammonium, dimethyl *cis*-9-octadecenyl-9, 12, 15-octadectrienyl ammonium, trimethyl monodocosanyl ammonium, methyl tridodecyl ammonium, and mixtures thereof.

Nonlimiting examples of antimicrobial quaternary ammonium compounds in group (b) of Formula (I) include the following: when $R_4$ is aryl alkyl or chloroaryl alkyl: benzalkonium which is a mixture of alkyl dimethyl benzyl ammonium compounds, wherein the alkyl group is a mixture of groups comprising from C-8 to C-18 hydrocarbons; or alkyl dimethyl dichlorobenzyl ammonium compounds. Preferred benzalkonium compounds are selected from the group consisting of tetradecyl dimethyl benzyl ammonium halide, hexadecyl (cetyl) dimethyl benzyl ammonium halide, and octadecyl (stearyl) dimethyl benzyl ammonium halide.

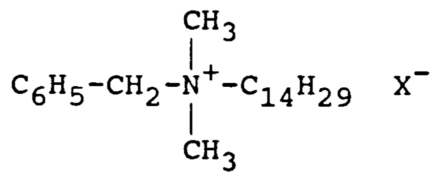

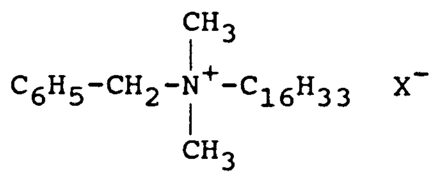

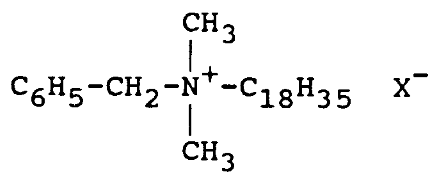

When $R_4$ is aryloxy alkyl, the antimicrobial quaternary ammonium compound may be dodecyl (lauryl) *beta*-phenoxyethyl dimethyl ammonium halide.

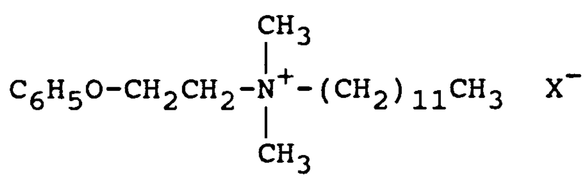

When $R_4$ is trialkyl ammonium alkyl, the antimicrobial quaternary ammonium compound may be trimethylammoniumhexyl trimethyl ammonium or trimethylammoniumdecyl trimethyl ammonium (decamethonium).

$(CH_3)_3N^+(CH_2)_6N^+(CH_3)_3\ 2X^-$
$(CH_3)_3N^+(CH_2)_{10}N^+(CH_3)_3\ 2X^-$

Preferably, the antimicrobial quaternary ammonium compound in group (b) is a mixture of benzalkonium chlorides.

Nonlimiting examples of antimicrobial quaternary ammonium compounds in group (c) of Formula (I) include the following: when $R_3$ and $R_4$ are aryl alkyl and monoalkylphenoxy alkyleneoxyalkyl, the antimicrobial quaternary ammonium compound may be (4-diisobutylphenoxy)ethoxyethyl dimethyl benzyl ammonium (benzethonium);

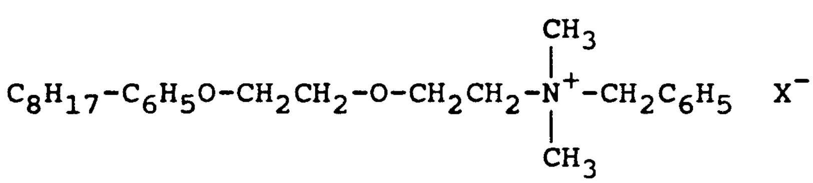

and when $R_3$ and $R_4$ are aryl alkyl and dialkylphenoxy alkyleneoxyalkyl, the antimicrobial quaternary ammonium compound may be [4-(diisobutyl)methylphenoxy]ethoxy-ethyldimethyl benzyl ammonium (methylbenzethonium).

$$C_8H_{17}-C_6H_4(CH_3)O-CH_2CH_2-O-CH_2CH_2-\overset{\overset{CH_3}{|}}{\underset{\underset{CH_3}{|}}{N^+}}-CH_2C_6H_5 \quad X^-$$

In a preferred embodiment, the antimicrobial quaternary ammonium compound in group (c) is [4-(diisobutyl)methylphenoxy)ethoxy-ethyl dimethyl benzyl ammonium (methylbenzethonium).

Nonlimiting examples of antimicrobial quaternary ammonium compounds in group (d) of Formula (I) include the following: lauryl pyridinium; cetylpyridinium, laurylisoquinolinium; laurylnicotinium; and lauryl-quinaldinium.

$C_5H_5N^+-C_{12}H_{25}\ X^-$
$C_5H_5N^+-C_{16}H_{33}\ X^-$
$C_9H_7N^+-C_{12}H_{25}\ X^-$
$HOOC-C_5H_4N^+-C_{12}H_{25}\ X^-$
$HOOC-C_9H_6N^+-C_{12}H_{25}\ X^-$

In a preferred embodiment, the antimicrobial quaternary ammonium compound in group (d) is selected from the group consisting of cetylpyridinium and laurylisoquinolinium, and mixtures thereof.

In Formula (I), $X^-$ represents a halogen. The term "halogen", as used herein, refers to the chemically related elements of fluorine, chlorine, bromine, and iodine. In a preferred embodiment, the halogen is selected from the group consisting of chlorine and bromine.

In a preferred embodiment, the antimicrobial quaternary ammonium compound in Formula (I) is selected from the group consisting of octadecyl trimethyl ammonium, dimethyl dioctadecyl ammonium, dimethyl *cis*-9-octadecenyl-9, 12, 15-octadectrienyl ammonium, trimethyl monodocosanyl ammonium, methyl tridodecyl ammonium, benzalkonium halide (a mixture of alkyl dimethyl benzyl ammonium compounds), [4-(diisobutyl)methylphenoxy]ethoxyethyl dimethyl benzyl ammonium (methylbenzethonium), cetylpyridinium, laurylisoquinolinium, and mixtures thereof. In a more preferred embodiment, the antimicrobial quaternary ammonium compound in Formula (I) is a mixture of benzalkonium chlorides.

The chlorinated or fluorinated hydrocarbon solvent utilized in the process of the present invention is an impregnating solvent which quickly solvates, swells, and penetrates the polyurethane polymeric matrix without dissolving or weakening the polyurethane polymer. The solvent must also be capable of substantially dissolving and allowing the molecules of the antimicrobial quaternary ammonium compound to enter and diffuse into the polyurethane matrix. The boiling point of the solvent should be sufficiently low so that it can be readily removed from the polyurethane medical device without causing degradation of the medical device or the antimicrobial quaternary ammonium compound. In general, the boiling point of the solvent will be from about 10° C. to about 70° C. and preferably from about 20° C. to about 42° C. Nonlimiting examples of chlorinated or fluorinated hydrocarbon solvents in the present invention include dichloromethane, chloroform, carbon tetrachloride, 1, 2-dichloroethane, 1, 1, 1-trichloroethane, 1, 1, 2-trichloroethane, and mixtures thereof. In a preferred embodiment, the chlorinated or fluorinated hydrocarbon solvent is selected from the group consisting of dichloromethane, 1, 2-dichloroethane, and 1, 1, 1-trichloroethane, and mixtures thereof. In a more preferred embodiment, the solvent is dichloromethane.

If necessary, a cosolvent may be added to the impregnating solvent to help dissolve the antimicrobial quaternary ammonium compound providing that it does not substantially interfere with the impregnation of the polyurethane medical device. Suitable cosolvents may be selected from the group of lower-alkanols consisting of methanol, ethanol, 1-propanol, 2-propanol, and mixtures thereof. Acetone may also be used as a cosolvent. The amount of cosolvent contemplated herein may be from about 1% to about 20%, by volume, based on the total volume of solvents.

In general, the amount of antimicrobial quaternary ammonium compound present in the chlorinated or fluorinated hydrocarbon solvent is such that, within a given period of treatment, as defined below, will cause sufficient quaternary ammonium compound to be impregnated into the medical device to provide prolonged antimicrobial activity in the environment of the device in vivo. By this is meant that a device treated in accordance with the invention will provide a zone of microbial inhibition within and on the surface of the device in the body. Typically, the antimicrobial quaternary ammonium compound will be present in the chlorinated or fluorinated hydrocarbon solvent in an amount from about 0.001% to about 5%, preferably

from about 0.1% to about 1%, and more preferably about 0.5%, by weight.

A preformed polyurethane medical device is treated with the impregnating solution by any suitable method such as by dipping, spraying or the like. The treatment may be carried out at any temperature up to the boiling point of the solvent with ambient temperature being preferred, for from about 10 seconds to about 30 minutes. Generally, longer treatment times would be utilized for thicker devices such as for example a test probe. For most devices, particularly various types of catheters, a treatment time of about 30 seconds to about 2 minutes, and more preferably about 1 minute is satisfactory. The excess impregnating solution is then removed and the treated device is dried. Any convenient method of drying may be used. Preferably, the device is oven dried to remove solvent at an elevated temperature, such as from about 50° C. to about 75° C., preferably 65° C., for about 10 minutes to about one hour, preferably about 20 minutes. If desired, the rate of drying may be enhanced by applying a partial vacuum or by using an air stream such as from a heat gun.

In a preferred embodiment, the preformed polyurethane medical device is washed with a chlorofluorohydrocarbon solvent to remove waxy substances therefrom prior to treatment with the impregnating solution. Such substances are typically present in polyurethane resins as lubricants and extrusion aides in the preparation of articles, such as medicinal devices, therefrom. During formation of articles, e.g., by molding or extruding, these substances may migrate to the surface of the device. Preferably, the chlorofluorohydrocarbon solvent is Freon TA™, a freon solvent containing acetone.

It is preferred to coat the devices treated in accordance with the subject invention with a layer of material which becomes lubricious when hydrated. For example, the polyurethane medical device may be coated with a coating layer solution which comprises a hydrophilic poly(N-vinyl lactam) in gamma-butyrolactone. The term "poly(N-vinyl lactam)" as used herein means physiologically acceptable homopolymers and copolymers of such N-vinyl lactams as N-vinylpyrrolidone, N-vinylbutyrolactam, N-vinylcaprolactam, and the like, and mixtures thereof.

In a preferred embodiment, the poly(N-vinyl lactam) is polyvinylpyrrolidone (PVP) homopolymer preferably having a weight average molecular weight of about 1,100,000. The coating layer solution comprises from about 1% to about 10%, preferably from about 2.5% to about 5%, by weight of PVP.

In general, the coating layer solution is prepared by dissolving the poly(N-vinyl lactam) in gamma-butyrolactone at room temperature. Any cosolvent such as one or more lower alkanols, is then added and the solution is contacted with the polyurethane medical device such as by dipping, spraying, or the like. The excess coating solution is then removed and the polyurethane medical device is oven dried to remove solvent at an elevated temperature, such as from about 60° C. to about 75° C., preferably 70° C., for about one to three hours, preferably two hours.

The polyurethane medical devices are sterilized by treatment with ethylene oxide. Antimicrobial compounds containing active hydrogen groups will be inactivated by this sterilization process while the antimicrobial quaternary ammonium compounds of the present invention will be unaffected.

The present invention is further illustrated by the following examples which are not intended to limit the effective scope of the claims. All parts and percentages in the examples and throughout the specification and claims are by weight of the final composition unless otherwise specified.

## Example 1

Antimicrobial impregnating solutions were prepared by dissolving benzalkonium chloride in methylene chloride at room temperature to concentrations of 1% and 3%, respectively. The medical devices coated were 15 cm lengths of polyurethane 14 gauge tubing extruded from Pellethane™ series 2363 polyurethane resin. A polyurethane tube was immersed in each of the above impregnating solutions for a contact time of one minute. During this period, the tubes expanded to about three times their original diameter. The tubes were then removed from the impregnating solution, drained and placed in a preheated oven at a temperature of about 65° C for twenty minutes to dry. During this drying period, the tubes contracted to their original diameter.

The resulting benzalkonium impregnated polyurethane tubes were then evaluated for antimicrobial activity by contacting the tubes with an agar dish inoculated with the bacteria Staphylococcus aureus. The polyurethane tube prepared using the 1% antimicrobial benzalkonium chloride solution produced a zone of inhibition 3.0 mm in diameter while the tube impregnated using the 3% solution produced a zone of inhibition 17.0 mm in diameter. These results show that the diameters of the zones of inhibition produced by the benzalkonium impregnated polyurethane tubes can be increased by increasing the concentration of benzalkonium chloride in the impregnating solution.

## Example 2

Two 7Fr gage catheter tubes extruded from Pellethane™ series 2363 polyurethane resin were impregnated with a 5% impregnating solution of benzalkonium chloride in methylene chloride according to the method described for Example 1. The tubes were immersed in normal saline solution for 24 hours and 7 days, respectively. The tubes were removed from the saline, dried, and tested against the Staphylococcus aureus as described in Example 1.

The catheter tube soaked in saline solution for 24 hours produced a zone of inhibition 7.0 mm in diameter while the catheter tube soaked in saline solution for 7 days produced a zone of inhibition 4.0 mm in diameter.

Accordingly, these results demonstrate that the polyurethane medical devices impregnated with antimicrobial compounds prepared according to the method of the present invention have prolonged antimicrobial activity even after one week of exposure to physiological saline.

## Example 3

Five 10g samples of polyurethane pellets, Pellethane™ grade 2363-90AR0120, were immersed in a 1% benzalkonium chloride solution in methylene chloride, for 10 minutes, 20 minutes, 30 minutes, 1 hour, and 24 hours, respectively. The relationship between time of impregnation and percentage weight increase of the polyurethane, after removal of the solvent, is shown in the following Table.

TABLE

| Impregnation Time | 10min. | 20min. | 30min. | 1hr | 24hr |
|---|---|---|---|---|---|
| Percentage Weight Increase | 0.57 | 0.70 | 0.96 | 1.47 | 2.47 |

Accordingly, these results show that the percentage weight increase of the polyurethane medical devices can be increased by increasing the contact time with the impregnation solution. Hence, as shown in the previous examples, the diameter of the zone of inhibition and the degree of prolonged antimicrobial activity can be increased by increasing the impregnation contact time.

The embodiments of the present invention described herein are merely exemplary and are not intended to limit the scope of the invention. Many variations and modifications may be made without departing from the spirit and scope of the invention. Applicant intends that all such modifications and variations are to be included within the scope of the invention as defined in the appended claims and their equivalents.

## Claims

1. A method for impregnating a preformed polyurethane medical device with an antimicrobial quaternary ammonium compound which comprises the steps of:
   (A) contacting said device with a solution containing said compound in a chlorinated or fluorinated hydrocarbon solvent thereby impregnating the device; and
   (B) removing the solvent;
   wherein the antimicrobial quaternary ammonium compound is represented by Formula (I):

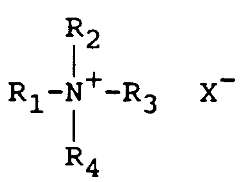

(I)

   including optically active isomeric forms thereof, wherein:
   (a) $R_1$, $R_2$, $R_3$, and $R_4$ are independently selected from the group consisting of straight- or branched-chain, saturated or unsaturated alkyl groups having from 1 to 20 carbon atoms; or
   (b) $R_1$, $R_2$, and $R_3$ are as defined above and $R_4$ is selected from the group consisting of aryl alkyl, chloroaryl alkyl, aryloxy alkyl, and trialkyl ammonium alkyl; or

(c) $R_1$ and $R_2$ are as defined above and $R_3$ and $R_4$ are independently selected from the group consisting of aryl alkyl, monoalkylaryloxy alkyleneoxy alkyl, and dialkylaryloxy alkyleneoxy alkyl; or

(d) $R_1$ is as defined above and $R_2$, $R_3$, $R_4$, and the nitrogen atom are members of a heterocyclic ring; and $X^-$ is a halogen.

**2.** The method according to claim 1, wherein the antimicrobial quaternary ammonium compound is selected from the group consisting of: octadecyl trimethyl ammonium; dimethyl dioctadecyl ammonium; dimethyl *cis*-9-octadecenyl-9, 12, 15-octadectrienyl ammonium; trimethyl monodocosanyl ammonium; methyl tridodecyl ammonium; benzalkonium (a mixture of alkyl dimethyl benzyl ammonium compounds); [4-(diisobutyl)methylphenoxy]ethoxy-ethyl dimethyl benzyl ammonium (methylbenzethonium); cetylpyridinium; laurylisoquinolinium; and mixtures thereof.

**3.** The method according to claim 2, wherein the antimicrobial quaternary ammonium compound is a mixture of benzalkonium chlorides.

**4.** The method according to claim 1, wherein the chlorinated or fluorinated hydrocarbon solvent is selected from the group consisting of dichloromethane, 1, 2-dichloroethane, 1, 1, 1-trichloroethane, and mixtures thereof.

**5.** The method according to claim 4, wherein the chlorinated or fluorinated hydrocarbon solvent is dichloromethane.

**6.** The method according to claim 1, wherein the antimicrobial quaternary ammonium compound is present in the chlorinated or fluorinated hydrocarbon solvent in an amount from about 0.001% to about 5%, by weight.

**7.** The method according to claim 6, wherein the antimicrobial quaternary ammonium compound is present in the chlorinated or fluorinated hydrocarbon solvent in an amount from about 0.1% to about 1%, by weight.

**8.** The method according to claim 1, wherein the polyurethane medical device is selected from the group consisting of intravenous and urinary catheters, test probes, peristaltic pump chambers, implant materials, arteriovenous shunts, gastroenteric feed tubes, endotracheal tubes, films for burn and wound dressings, sponges for wound cleansing, and condoms.

**9.** The method according to claim 1, wherein the polyurethane medical device is washed with a chlorofluorohydrocarbon solvent to remove waxy substances therefrom prior to the step of contacting said device with the impregnating solution.

**10.** A polyurethane medical device impregnated with an antimicrobial compound prepared by the method of claim 1.

European Patent Office

# EUROPEAN SEARCH REPORT

Application Number

EP 92 10 7452

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y,D | EP-A-0 405 284 (HERCULES INCORPORATED)<br>* column 3, line 1 *<br>* column 3, line 12 - line 30 *<br>* column 4, line 11 - line 13 *<br>* column 4, line 46; claims *<br>--- | 1-10 | A61L29/00<br>A61L27/00<br>A61L31/00<br>A61L15/44<br>A61L15/26 |
| Y | DATABASE WPIL<br>Section Ch, Week 8525,<br>Derwent Publications Ltd., London, GB;<br>Class A96, AN 85-148934<br>& JP-A-60 080 458 (UNITIKA)<br>* abstract *<br>--- | 1-10 | |
| A | US-A-4 713 402 (D. SOLOMON)<br>--- | | |
| A,D | US-A-4 605 564 (J. KULLA)<br>--- | | |
| A | WO-A-8 904 682 (COLORADO BIOMEDICAL INCORPORATED)<br>----- | | |
| | | | TECHNICAL FIELDS SEARCHED (Int. Cl.5 ) |
| | | | A61L |

The present search report has been drawn up for all claims

| Place of search | Date of completion of the search | Examiner |
|---|---|---|
| THE HAGUE | 06 OCTOBER 1992 | G.COUSINS-VAN STEEN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)